# EUROPEAN PATENT APPLICATION

(11) **EP 4 478 369 A1**
(43) Date of publication of application: **18.12.2024**
(21) Application number: 23753118.1
(22) Date of filing: 07.02.2023
(51) Int. Cl.: G16B 30/00, G16B 40/20, C12Q 1/6869, G06N 20/00

(54) **METHOD AND DEVICE FOR ANALYZING VARIANT TYPE OF ORGANISM**

(30) Priority: 08.02.2022 KR 20220016490
(71) Applicant: Seegene, Inc., Seoul 05548 (KR)
(72) Inventor: KIM, Hyunho, Seoul 05544 (KR); JANG, Jongha, Seoul 05546 (KR)
(74) Representative: Deambrogi, Edgardo
(86) International application number: PCT/KR2023/001756
(87) International publication number: WO 2023/153770

(57) **Abstract**

Disclosed is a method for analyzing a variant type of an organism, performed by a computing device. The method may include: preparing a variant classification model trained with a plurality of datasets, each of the plurality of datasets including a mutation information including at least one mutation identifier and a variant type labeled thereto; providing a plurality of mutation information to the variant classification model to obtain variant types for each of the plurality of mutation information; and calculating a contribution of said at least one mutation identifier to determining each of the variant types obtained, based on the plurality of mutation information and each of the variant types obtained. A representative figure may be FIG. 2.

## Description

### [Technical Field]

The present disclosure relates to a method for analyzing an organism, and more particularly, to a method and a device for analyzing a variant type of an organism.

### [Background Art]

As the next generation sequencing (NGS) technology has been developed and the set of a large number of base sequences can be processed in parallel, the base sequence analysis technology is utilized in the form applicable to various fields.

As the center of therapeutic medicine is transformed into preventive medicine, with the development of molecular genetic technology, the technology that searches base sequences specific to a specific living organism group is particularly spotlighted in the field of in vitro diagnostic (IVD) (especially molecular diagnosis field).

Various variants are rapidly generated in the specific living organism groups (e.g., viruses), so the specific living organism groups are continuously changed to a type suitable for survival. According to the frequent appearance of the variants, the development of new diagnostic kits and the development of new vaccines to target, diagnose and treat variants should be made quickly. The variants can be generated as part of the base sequence of the corresponding living organism group is changed. For example, SARS-COV-2, a virus that causes COVID-19, is reported to be generated as a variant that selectively deletes small pieces of the base sequence in repeated evolutionary patterns.

Currently, international institutions such as WHO are monitoring newly generated variants of these viruses and providing variant identification information or variant cause information. As a result of this monitoring, the base sequences of the newly generated variants are released, allowing you to see what amino acids have mutation within a new variant. As a result, with the emergence of new variants, research and development for diagnosing and treating new variants is being conducted subsequently.

In order to search for base sequence or amino acid specific to specific variants in a specific living organism group, it is necessary to figure out what a base sequence or amino acid is specific for a specific variant. As the type and number of variants newly added to the database increase, not only many computing resources are unnecessarily consumed to analyze and search for a base sequence or amino acid specific to a specific variant, but also takes a long time for such analysis and search.

Korean Patent Unexamined Publication No. 2017-0046315 presents a technology of aligning and comparing base sequences of a mother model and a father model in a known reference sequence in order to search for a single base sequence variant of each of the mother model and the father model obtained through next generation sequencing.

### [Disclosure]

### [Technical Problem]

The present disclosure is contrived in response to the above-described background art, and has been made in an effort to obtain mutations specific to a variant type of a target analyte.

### [Technical Solution]

Disclosed is a method for analyzing a variant type of an organism, performed by a computing device according to an embodiment of the present disclosure. The method may include: preparing a variant classification model trained with a plurality of datasets, each of the plurality of datasets including a mutation information including at least one mutation identifier and a variant type labeled thereto; providing a plurality of mutation information to the variant classification model to obtain variant types for each of the plurality of mutation information; and calculating a contribution of said at least one mutation identifier to determining each of the variant types obtained, based on the plurality of mutation information and each of the variant types obtained.

In an embodiment, the analyzing the variant type may include the obtaining of the variant type and the calculating of the contribution.

In an embodiment, the variant classification model may be a multi class classification model pre-trained based on at least one of Random forest, K-nearest neighbor (KNN), Naive bayes, or multi-layer perceptron (MLP), and here, one class may correspond to one variant type.

In an embodiment, an amino acid which is mutated and/or not mutated may be identified from the at least one mutation identifier.

In an embodiment, the mutation information may be vectorized into a dimension corresponding to a count of the one or more mutation identifiers.

In an embodiment, the plurality of datasets used for training may be the datasets subjected to a dimension reduction algorithm to reduce a count of a plurality of the mutation identifiers included in the mutation information.

In an embodiment, the plurality of datasets used for the training may be datasets subjected to a filtering algorithm which removes one or more datasets based on a count of mutation occurrences.

In an embodiment, the contribution may be calculated based on a difference found between the plurality of mutation information and a difference between the variant types obtained in the variant classification model due to the found difference.

In an embodiment, the contribution may be calculated using explainable AI (artificial intelligence) operating a feature importance of each of the mutation identifiers for each of the variant types in the variant classification model.

In an embodiment, the method may further include: generating perturbation input data by changing at least one of the plurality of mutation information; and providing the perturbation input data to the variant classification model to obtain a variant type therefrom; and the input perturbation input data and the variant type obtained therefrom may be additionally used for calculating the contribution.

In an embodiment, the method may further include calculating, based on the calculated contribution, a specificity ranking of the plurality of mutation identifiers for each of the variant types obtained.

In an embodiment, the calculating the contribution may include calculating the contribution of each of the plurality of mutation identifiers for each variant type obtained such that each of the plurality of mutation identifiers included in the mutation information is allowed to comprise a positive influence value or a negative influence value.

According to an embodiment of the present disclosure, disclosed is a computing device including: a memory comprising at least one instruction; and a processor. By the computing device, as the at least one instruction is executed by the processor, a variant classification model trained with a plurality of datasets may be prepared, wherein each of the plurality of datasets including a mutation information including at least one mutation identifier and a variant type labeled thereto; a plurality of mutation information may be provided to the variant classification model to obtain variant types for the each of the plurality of mutation information; and a contribution of said at least one mutation identifier to determining each of the variant types obtained may be calculated, based on the plurality of mutation information and each of the variant types obtained.

A computer-readable recording medium on which a computer program according to an embodiment of the present disclosure is stored may be programmed to perform each step included in the method according to the present disclosure.

### [Advantageous Effects]

A method and a device according to an embodiment of the present disclosure can obtain mutations specific to a variant type of a target analyte in an efficient scheme.

### [Description of Drawings]

FIG. 1 schematically illustrates a block diagram of a computing device according to an embodiment of the present disclosure.
FIG. 2 illustrates a flowchart of calculating a contribution of mutation identifiers for variant types according to an embodiment of the present disclosure.
FIG. 3 exemplarily illustrates a conceptual view for a process of training a variant classification model according to an embodiment of the present disclosure.
FIG. 4 exemplarily illustrates a conceptual view for an operation of a variant classification model trained according to an embodiment of the present disclosure.
FIG. 5 exemplarily illustrates a conceptual view of calculating a contribution of AA mutation identifiers for variant types by using the variant classification model according to an embodiment of the present disclosure.
FIG. 6 illustrates an exemplary data structure including a contribution of AA mutation identifiers for variant types according to an embodiment of the present disclosure.
FIG. 7 exemplarily illustrates a data structure for visually showing the contribution of AA mutation identifiers for variant types according to an embodiment of the present disclosure.
FIG. 8 exemplarily illustrates a data structure for visually showing the contribution of AA mutation identifiers for variant types according to an embodiment of the present disclosure.
FIG. 9 is a schematic diagram of a computing environment according to an embodiment of the present disclosure.

### [Mode for Invention]

Various exemplary embodiments and/or aspects will be now disclosed with reference to drawings. In the following description, for the purpose of a description, multiple detailed matters will be disclosed in order to help comprehensive appreciation of one or more aspects. In describing the present disclosure, a detailed description of known function or constitutions will be omitted if it is determined that it unnecessarily makes the gist of the present disclosure unclear. Furthermore, the terms or words used in this specification and the claims should be interpreted in line with the technical concept of the present disclosure, in accordance with the principle that the inventor can define the concepts of appropriate terms in order to describe their invention in the best possible manner.

"Component", "module", "system", "unit" and the like which are terms used in the specification refer to a computer-related entity, hardware, firmware, software, and a combination of the software and the hardware, or execution of the software. For example, the component may be a processing process executed on a processor, the processor, an object, an execution thread, a program, and/or a computer, but is not limited thereto. For example, both an application executed in a computing device and the computing device may be the components. One or more components may reside within the processor and/or a thread of execution. One component may be localized in one computer. One component may be distributed between two or more computers. Further, the components may be executed by various computer-readable media having various data structures, which are stored therein. The components may perform communication through local and/or remote processing according to a signal (for example, data transmitted from another system through a network such as the Internet through data and/or a signal from one component that interacts with other components in a local system and a distribution system) having one or more data packets, for example.

The term "or" is intended to mean not exclusive "or" but inclusive "or". That is, when not separately specified or not clear in terms of a context, a sentence "X uses A or B" is intended to mean one of the natural inclusive replacements. That is, the sentence "X uses A or B" may be applied to any of the case where X uses A, the case where X uses B, or the case where X uses both A and B. Further, it should be understood that the term "and/or" used in this specification designates and includes all available combinations of one or more items among enumerated related items.

It should be appreciated that the term "include", "comprise", and/or "including", "comprising" means presence of corresponding features and/or components. However, it should be appreciated that the term "include", "comprises" and/or "including", "comprising" means that presence or addition of one or more other features, components, and/or a group thereof is not excluded. Further, when not separately specified or it is not clear in terms of the context that a singular form is indicated, it should be construed that the singular form generally means "one or more" in this specification and the claims.

The term "at least one of A or B" or "at least one of A and B" should be interpreted to mean "a case including only A", "a case including only B", and "a case in which A and B are combined".

Those skilled in the art need to recognize that various illustrative logical blocks, configurations, modules, circuits, means, logic, and algorithm steps described in connection with the exemplary embodiments disclosed herein may be additionally implemented as electronic hardware, computer software, or combinations of both sides. To clearly illustrate the interchangeability of hardware and software, various illustrative components, blocks, configurations, means, logic, modules, circuits, and steps have been described above generally in terms of their functionalities. Whether the functionalities are implemented as the hardware or software depends on a specific application and design restrictions given to an entire system. Skilled artisans may implement the described functionalities in various ways for each specific application. However, such implementation decisions should not be interpreted as causing a departure from the scope of the present disclosure.

The description of the presented embodiments is provided so that those skilled in the art of the present disclosure use or implement the present disclosure. Various modifications to the exemplary embodiments will be apparent to those skilled in the art. Generic principles defined herein may be applied to other embodiments without departing from the scope of the present disclosure. Therefore, the present disclosure is not limited to the exemplary embodiments presented herein. The present disclosure should be analyzed within the widest range which is coherent with the principles and new features presented herein.

In the present disclosure terms represented by N-th such as first, second, or third are used for distinguishing at least one entity. For example, entities expressed as first and second may be the same as each other or different from each other.

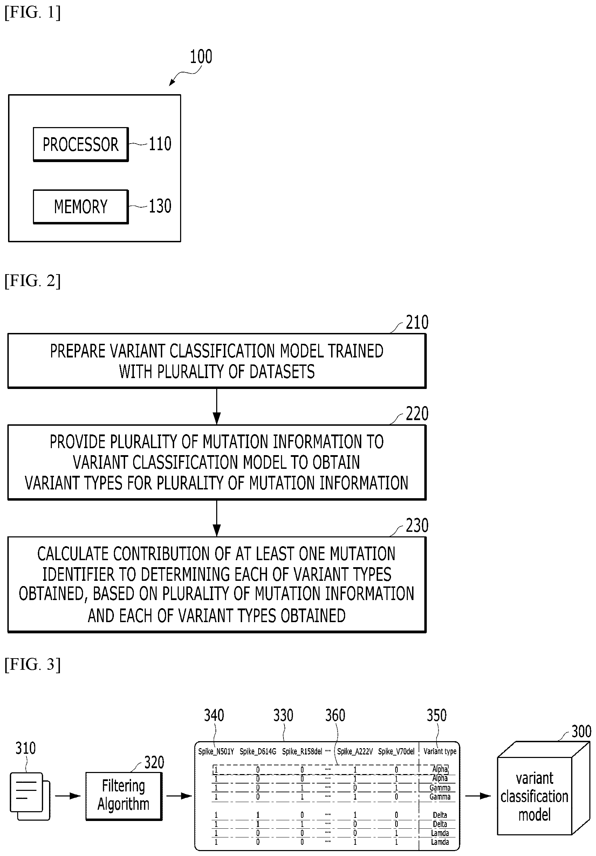

FIG. 1 schematically illustrates a block diagram of a computing device 100 according to an embodiment of the present disclosure.

According to the embodiment of the present disclosure, the computing device 100 may include a processor 110 and a memory 130.

A configuration of the computing device 100 illustrated in FIG. 1 is only an example shown through simplification. In an embodiment of the present disclosure, the computing device 100 may include other components for performing a computing environment of the computing device 100, and only some of the disclosed components may also constitute the computing device 100.

In the present disclosure, the computing device 100 may mean a node constituting a system for implementing embodiments of the present disclosure. The computing device 100 may mean any type of user terminal or any type of server. The components of the computing device 100 may be exemplary and some components may be excluded, or an additional component may be included in the computing device 100. As an example, when the computing device 100 includes a terminal, an output unit (not illustrated) and an input unit (not illustrated) may be included in a range of the computing device 100.

The computing device 100 in the present disclosure may perform technical features according to embodiments of the present disclosure to be described below. The computing device 100 may train a variant classification model. The computing device 100 may calculate contributions of mutation identifiers by using the trained variant classification model.

The "target analyte" used in the present disclosure may include any form of organisms to be analyzed, obtained, or detected. For example, the organism may mean a living thing or an organism which belongs to one genus, species, subspecies, subtype, genotype, serotype, strain, isolate, or cultivar. In the present disclosure, the living thing and the organism may be used interchangeably with each other.

As a non-limiting example, the organism may include prokaryotic cells (e.g., Mycoplasma pneumoniae, Chlamydophila pneumoniae, Legionella pneumophila, Haemophilus influenzae, Streptococcus pneumoniae, Bordetella pertussis, Bordetella parapertussis, Neisseria meningitidis, Listeria monocytogenes, Streptococcus agalactiae, Campylobacter, Clostridium difficile, Clostridium perfringens, Salmonella, Escherichia coli, Shigella, Vibrio, Yersinia enterocolitica, Aeromonas, Chlamydia trachomatis, Neisseria gonorrhoeae, Trichomonas vaginalis, Mycoplasma hominis, Mycoplasma genitalium, Ureaplasma urealyticum, Ureaplasma parvum, Mycobacterium tuberculosis, Treponema pallidum, Candida, Mobiluncus, Megasphaera, Lacto spp., Mycoplasma genitalium, Clostridium difficile, Helicobacter Pylori, ClariR, CPE, Group B Streptococcus, Enterobacter cloacae complex, Proteus mirabilis, Klebsiella aerogenes, Pseudomonas aeruginosa, Klebsiella oxytoca, Serratia marcescens, Klebsiella pneumoniae, Actinomycetaceae actinotignum, Enterococcus faecium Staphylococcus epidermidis, Enterococcus faecalis, Staphylococcus saprophyticus, Staphylococcus aureus, Acinetobacter baumannii, Morganella morganii, Aerococcus urinae, Pantoea aglomerans, Citrobacter Freundii, Providencia stuartii, Citrobacter koseri, Streptococcus anginosus, Trichophyton mentagrophytes complex, Microsporum spp., Trichophyton rubrum, Epidermophyton floccosum, Trichophyton tonsurans), eukaryotic cells (e.g., protozoa and parasitic animals, fungi, yeast, higher plants, lower animals, and higher animals such as mammals and humans), virus, or viroid. Among the eukaryotic cells, parasite may include, for example, Giardia lamblia, Entamoeba histolytica, Cryptosporidium, Blastocystis hominis, Dientamoeba fragilis, Cyclospora cayetanensis, stercoralis, trichiura, hymenolepis, Necator americanus, Enterobius vermicularis, Taenia spp., Ancylostoma duodenale, Ascaris lumbricoides, Enterocytozoon spp./Encephalitozoon spp. The virus may include, for example, influenza A virus (flue A), influenza B virus (flu B), Respiratory syncytial virus A (RSV A), Respiratory syncytial virus B (RSV B), Covid-19 virus, Parine Fluenza Virus 1 (PIV 1), Parine Fluenza Virus 2 (PIV 2), Parine Fluenza Virus 3 (PIV 3), Parine Fluenza Virus 4 (PIV 4), metapneumovirus (MPV), human enterovirus (HEV), Human Boca Virus (HBOV), Human Rhinobirus (HRV), Coronavirus and Adenovirus causing respiratory diseases, and Norovirus, Rotavirus, Adenovirus, Astrovirus, and Sapo Virus causing gastrointestinal diseases. As another example, the virus may include human papillomavirus (HPV), Middle East respiratory syndrome-related coronavirus (MERS-CoV), Dengue virus, Herpes simplex virus (HSV), Human herpes virus (HHV), Epstein-Barr virus (EMV), Varicella zoster virus (VZV), Cytomegalovirus (CMV), HIV, Parvovirus B19, Parechovirus, Mumps, Dengue virus, Chikungunya virus, Zika virus, West Nile virus, hepatitis virus, and poliovirus. The target analyte may be GBS serotype, Bacterial colony, or v600e. The target analyte in the present disclosure may include various analysis targets such as bacteria in addition to the virus, and may also be a specific site of genes cut by using a CRISPR technology, and a range of the target analyte is not limited to the examples.

In an embodiment, the target analyte of the present disclosure is an organism in which a plurality of variants are generated, and an amino acid sequence specific to each variant of the target analyte may be identified based on a technique according to an embodiment of the present disclosure.

In an embodiment, the processor 110 may be constituted by at least one core and may include processors for data analysis and/or processing, which include a central processing unit (CPU), a general purpose graphics processing unit (GPGPU), a tensor processing unit (TPU), and the like of the computing device 100. The processor 110 reads the computer program stored in the memory 130 to obtain, by using a pre-trained variant classification model according to an embodiment of the present disclosure, a classification result for the variant type from input data in which at least a part of mutation information is changed. The above-described input data may include, for example, perturbation input data. The processor 110 reads the computer program stored in the memory 130 to calculate the contribution of each of the plurality of mutation identifiers of the mutation information for each of the plurality of variant types according to an embodiment of the present disclosure.

According to an embodiment of the present disclosure, the processor 110 may perform an operation for learning the neural network. The processor 110 may perform calculations for learning the neural network, such as processing of input data for learning in deep learning (DL), extracting a feature in the input data, calculating an error, updating a weight of the neural network using backpropagation, and the like. At least one of the CPU, GPGPU, and TPU of the processor 110 may process learning of a network function. For example, both the CPU and the GPGPU may process the learning of the network function and data classification using the network function. Further, in an embodiment of the present disclosure, processors of the plurality of computing devices may be used together to process the learning of the network function and the data classification using the network function. Further, the computer program executed in the computing device according to an embodiment of the present disclosure may be a CPU, GPGPU, or TPU executable program.

According to an embodiment of the present disclosure, the memory 130 may store any type of information generated or determined by the processor 110 or any type of information received by the computing device 100. According to an embodiment of the present disclosure, the memory 130 may be a storage medium that stores computer software which allows the processor 110 to perform the operations according to the embodiments of the present disclosure. Therefore, the memory 130 may mean computer-readable media for storing software codes required for performing the embodiments of the present disclosure, data which become execution targets of the codes, and execution results of the codes.

According to an embodiment of the present disclosure, the memory 130 may mean any type of storage medium. For example, the memory 130 may include at least one type of storage medium of a flash memory type storage medium, a hard disk type storage medium, a multimedia card micro type storage medium, a card type memory (for example, an SD or XD memory, or the like), a random access memory (RAM), a static random access memory (SRAM), a read-only memory (ROM), an electrically erasable programmable read-only memory (EEPROM), a programmable read-only memory (PROM), a magnetic memory, a magnetic disk, and an optical disk. The computing device 100 may operate in connection with a web storage performing a storing function of the memory 130 on the Internet. The description of the memory is just an example and the memory 130 used in the present disclosure is not limited to the examples.

The computing device 100 in the present disclosure may further include a communication unit (not illustrated). The communication unit may be configured regardless of communication modes such as wired and wireless modes and constituted by various communication networks including a personal area network (PAN), a wide area network (WAN), and the like. Further, the network unit 150 may operate based on known World Wide Web (WWW) and may adopt a wireless transmission technology used for short-distance communication, such as infrared data association (IrDA) or Bluetooth.

The computing device 100 in the present disclosure may include any type of user terminal and/or any type of server. The user terminal may include any type of terminal which is capable of interacting with the server or another computing device. The user terminal may include, for example, a mobile phone, a smartphone, a laptop computer, personal digital assistants (PDA), a slate PC, a tablet PC, and an Ultrabook.

The server may include, for example, any type of computer system or computing device such as a microprocessor, a mainframe computer, a digital processor, a portable device, and a device controller.

In an additional embodiment, the server may mean an entity storing and managing base sequence information or gene information. The server may include a storage unit (not illustrated) for storing the base sequence information, the gene information, and/or index information, and the storage unit may be included in the server or may be present under the management of the server. As another example, the storage unit may also be present outside the server, and implemented in a form which is capable of communicating with the server. In this case, the storage unit may be managed and controlled by another external server different from the server.

FIG. 2 illustrates a flowchart of calculating a contribution of mutation identifiers for variant types according to an embodiment of the present disclosure.

In an embodiment, the steps illustrated in FIG. 2 may be performed by the computing device 100. In an additional embodiment, like a scheme in which some of the steps illustrated in FIG. 2 are performed by the user terminal and some other steps are performed by the server, the steps in FIG. 2 may also be implemented by a plurality of entities.

As illustrated in FIG. 2, the computing device 100 may provide a pre-trained variant classification model from training data in which the variant type of the organism is labeled with the mutation information in the organism (210).

In the present disclosure, the variant type may mean information for identifying what a variant corresponding to the target analyte is. For example, a variant type of a virus such as SARS-CoV-2 may include alpha variant, beta variant, and/or gamma variant.

In the present disclosure, the mutation information may be information representing mutation at an amino acid level or mutation at a nucleic acid level. The mutation information may include amino acid (AA) mutation information. For convenience of description, hereinafter, the AA mutation information will be used as an example for the mutation information.

In the present disclosure, the AA mutation information may mean any type of information for identifying amino acids in which mutation occurs in the variant of the target analyte. For example, the AA mutation information may include information representing how the amino acid in which the mutation occurs in the variant of the target analyte is changed. As another example, the AA mutation information may be obtained from a public data server such as GISAID or NCBI, for example, and the AA mutation information may include information representing whether a specific amino acid for each variant type is mutated. As yet another example, the AA mutation information may include information representing whether mutation occurs for each AA mutation identifier mapped to the variant type. In an embodiment, the AA mutation information may include at least one AA mutation identifier. In an embodiment, the AA mutation information may include information indicating whether the mutation occurs in at least one AA mutation identifier for each variant.

In the present disclosure, the mutation identifier is used for identifying mutation. The mutation identifier may include the AA mutation identifier. For convenience of description, hereinafter, the AA mutation identifier will be used as an example for the mutation identifier. In an embodiment, the AA mutation identifier may mean an indicator for identifying what amino acid is changed and how the amino acid is changed. For example, the AA mutation identifier may include location information of the corresponding amino acid, and identification information representing how the corresponding amino acid is mutated in a specific protein. The AA mutation identifier may be included in the AA mutation information. For example, an AA mutation identifier expressed as "spike_N501Y" may identify a mutation in which 501^{st} N amino acid of spike protein is changed to Y amino acid. As another example, an AA mutation identifier expressed as "spike_R158del" is an identifier representing mutation in which 158^{th} R amino acid of the spike protein is deleted. In an embodiment, a value of the AA mutation identifier may include a binary value. For example, a value of 1 may be allocated in order to express that the mutation of the AA mutation identifier expressed as "spike_N501Y" occurs, and a value of 0 may be allocated in order to express that the mutation of the AA mutation identifier does not occur.

In an embodiment of the present disclosure, the variant classification model may be trained based on training data including input data corresponding to the AA mutation information of the target analyte and ground-truth data corresponding to the variant type of the target analyte. For example, the variant classification model may generate output data in response to input data including a vector in which whether each of the plurality of AA mutation identifiers is mutated is encoded. Here, the output data may include at least one variant type and a score therefor. For example, the output data may include a probability value for each of classes corresponding to a plurality of variant types. In such an example, the variant classification model may be a multi-class classification model, and one class and one variant type may correspond to each other. As an example, when 29 variants are present for the SARS-CoV-2 virus, the variant classification model may generate output data for a total of 29 classes.

In an embodiment of the present disclosure, the computing device 100 may obtain a classification result for the variant type from a plurality of predetermined mutation information (220).

In an embodiment of the present disclosure, as the computing device 100 analyzes the classification result for the variant type generated from the plurality of predetermined mutation information, contributions of a plurality of mutation identifiers included in the plurality of mutation information may be calculated.

In an embodiment of the present disclosure, the computing device 100 may obtain output data including a corresponding variant type by inputting perturbation input data in which at least some of the AA mutation information is changed into the variant classification model. In an embodiment, the perturbation input data may include input data in which whether the mutation occurs in the AA mutation identifier included in the AA mutation information or not is changed. For example, the perturbation input data may be generated by changing the value allocated to the AA mutation identifier included in the AA mutation information from 1 to 0 or from 0 to 1.

In an embodiment, the classification result output from the variant classification model as the perturbation input data is input into the variant classification model may include at least one variant type corresponding to the perturbation input data and a score corresponding to the at least one variant type.

In an embodiment of the present disclosure, the computing device 100 may calculate the contributions of the plurality of mutation identifiers included in the plurality of mutation information for each of the plurality of variant types, at least partially based on the information input into the variant classification model and the obtained classification result (230).

In an embodiment, the computing device 100 may calculate the contributions of the plurality of mutation identifiers included in the plurality of mutation information based on outputs obtained in response to the plurality of mutation information input into the variant classification model.

As an example, the information input into the variant classification model may include perturbation input data in which at least part of the plurality of mutation identifiers included in the plurality of mutation information are changed.

In an embodiment, the computing device 100 may analyze output data output in response to the perturbation input data in which a value of input data corresponding to the AA mutation information is changed by using the variant classification model to calculate a feature importance of each of the AA mutation identifiers of the AA mutation information.

In an embodiment, which AA mutation identifier plays an important role in determining a specific variant type among the AA mutation identifiers of the AA mutation information may be determined based on the calculated feature importance.

The contribution in the present disclosure may mean, for example, a quantitative value representing whether each of the AA mutation identifiers is used as an important factor in determining the specific variant type. As another example, the contribution may mean a quantitative value at which each of the AA mutation identifiers contributes to determining the specific variant type. As another example, the contribution may also mean a quantitative value at which each of the AA mutation identifiers specifically contributes to determining the specific variant type. In this case, at the time of calculating the contribution for the specific variant type, a contribution in another variant type may be additionally considered.

In an embodiment, the contribution may be calculated differently according to each of the AA mutation identifiers and according to each of the variant types. As an example, when an AA mutation identifier expressed as "spike_D501G" is relatively less found or not found in samples corresponding to Alpha variant (that is, when there is a high possibility that mutation of the AA mutation identifier expressed as "spike_D501G" will not be present in the Alpha variant), the contribution of the AA mutation identifier may be calculated as a low value for the Alpha variant. As another example, when an AA mutation identifier expressed as "spike_D501G" is relatively more found in samples corresponding to Delta variant (that is, when there is a high possibility that mutation of the AA mutation identifier expressed as "spike_D501G" will be present in the Delta variant), the contribution of the AA mutation identifier may be calculated as a high value for the Delta variant.

In an embodiment of the present disclosure, the computing device 100 may calculate a specificity ranking of the plurality of mutation identifiers for each of the plurality of variant types, from the calculated contribution. As an example, the plurality of mutation identifiers may be output in an order in which the specificity is the highest. As another example, a plurality of mutation identifiers in which the specificity is more than a predetermined threshold may also be output.

FIG. 3 exemplarily illustrates a conceptual view for a process of training a variant classification model according to an embodiment of the present disclosure.

In an embodiment, a process of training the variant classification model 300 illustrated in FIG. 3 may be performed by the computing device 100. In an additional embodiment, like a scheme in which a part of the process of training the variant classification model 300 illustrated in FIG. 3 is performed by the user terminal and the other part is performed by the server, the steps in FIG. 3 may also be implemented by a plurality of entities.

In an embodiment of the present disclosure, the variant classification model 300 may include a multi class classification model pre-trained based on at least one of Random forest, K-nearest neighbor (KNN), Naive bayes, or multi-layer perceptron (MLP). Here, one class may correspond to one variant type.

In this specification, a model may refer to any form of computer program operating based on a network function, an artificial neural network, and/or a neural network. Throughout the present specification, a computation model, the neural network, a network function, and the neural network may be used interchangeably with each other. The neural network may be generally constituted by an aggregate of calculation units which are mutually connected to each other, which may be called nodes. The nodes may also be called neurons. The neural network is configured to include one or more nodes. The nodes (alternatively, neurons) constituting the neural networks may be connected to each other by one or more links.

In the neural network, one or more nodes connected through the link may relatively form the relationship between an input node and an output node. Concepts of the input node and the output node are relative and a predetermined node which has the output node relationship with respect to one node may have the input node relationship in the relationship with another node and vice versa. As described above, the relationship of the input node to the output node may be generated based on the link. One or more output nodes may be connected to one input node through the link and vice versa.

In the relationship of the input node and the output node connected through one link, a value of data of the output node may be determined based on data input in the input node. Here, a link connecting the input node and the output node to each other may have a weight. The weight may be variable and the weight is variable by a user or an algorithm in order for the neural network to perform a desired function. For example, when one or more input nodes are mutually connected to one output node by the respective links, the output node may determine an output node value based on values input in the input nodes connected with the output node and the weights set in the links corresponding to the respective input nodes.

As described above, in the neural network, one or more nodes are connected to each other through one or more links to form a relationship of the input node and output node in the neural network. A characteristic of the neural network may be determined according to the number of nodes, the number of links, correlations between the nodes and the links, and values of the weights granted to the respective links in the neural network. For example, when the same number of nodes and links exist and there are two neural networks in which the weight values of the links are different from each other, it may be recognized that two neural networks are different from each other.

The neural network may be constituted by a set of one or more nodes. A subset of the nodes constituting the neural network may constitute a layer. Some of the nodes constituting the neural network may constitute one layer based on the distances from the initial input node. For example, a set of nodes of which distance from the initial input node is n may constitute n layers. The distance from the initial input node may be defined by the minimum number of links which should be passed through for reaching the corresponding node from the initial input node. However, a definition of the layer is predetermined for description and the order of the layer in the neural network may be defined by a method different from the aforementioned method. For example, the layers of the nodes may be defined by the distance from a final output node.

The initial input node may mean one or more nodes in which data is directly input without passing through the links in the relationships with other nodes among the nodes in the neural network. Alternatively, in the neural network, in the relationship between the nodes based on the link, the initial input node may mean nodes which do not have other input nodes connected through the links. Similarly thereto, the final output node may mean one or more nodes which do not have the output node in the relationship with other nodes among the nodes in the neural network. Further, a hidden node may mean nodes constituting the neural network other than the initial input node and the final output node.

In the neural network according to an exemplary embodiment of the present disclosure, the number of nodes of the input layer may be the same as the number of nodes of the output layer, and the neural network may be a neural network of a type in which the number of nodes decreases and then, increases again from the input layer to the hidden layer. Further, in the neural network according to another exemplary embodiment of the present disclosure, the number of nodes of the input layer may be smaller than the number of nodes of the output layer, and the neural network may be a neural network of a type in which the number of nodes decreases from the input layer to the hidden layer. Further, in the neural network according to yet another exemplary embodiment of the present disclosure, the number of nodes of the input layer may be larger than the number of nodes of the output layer, and the neural network may be a neural network of a type in which the number of nodes increases from the input layer to the hidden layer. The neural network according to still yet another exemplary embodiment of the present disclosure may be a neural network of a type in which the neural networks are combined.

A deep neural network (DNN) may refer to a neural network that includes a plurality of hidden layers in addition to the input and output layers. When the deep neural network is used, the latent structures of data may be determined. That is, latent structures of photos, text, video, voice, and music (e.g., what objects are in the photo, what the content and feelings of the text are, what the content and feelings of the voice are) may be determined. The deep neural network may include a convolutional neural network (CNN), a recurrent neural network (RNN), an auto encoder, generative adversarial networks (GAN), a restricted Boltzmann machine (RBM), a deep belief network (DBN), a Q network, a U network, a Siam network, a Generative Adversarial Network (GAN), and the like. The description of the deep neural network described above is just an example and the present disclosure is not limited thereto.

The neural network may be learned in at least one scheme of supervised learning, unsupervised learning, semi supervised learning, or reinforcement learning. The learning of the neural network may be a process in which the neural network applies knowledge for performing a specific operation to the neural network.

The neural network may be learned in a direction to minimize errors of an output. The learning of the neural network is a process of repeatedly inputting learning data into the neural network and calculating the output of the neural network for the learning data and the error of a target and back-propagating the errors of the neural network from the output layer of the neural network toward the input layer in a direction to reduce the errors to update the weight of each node of the neural network. In the case of the supervised learning, the learning data labeled with a correct answer is used for each learning data (i.e., the labeled learning data) and in the case of the unsupervised learning, the correct answer may not be labeled in each learning data. That is, for example, the learning data in the case of the supervised learning related to the data classification may be data in which category is labeled in each learning data. The labeled learning data is input to the neural network, and the error may be calculated by comparing the output (category) of the neural network with the label of the learning data. As another example, in the case of the unsupervised learning related to the data classification, the learning data as the input is compared with the output of the neural network to calculate the error. The calculated error is back-propagated in a reverse direction (i.e., a direction from the output layer toward the input layer) in the neural network and connection weights of respective nodes of each layer of the neural network may be updated according to the back propagation. A variation amount of the updated connection weight of each node may be determined according to a learning rate. Calculation of the neural network for the input data and the back-propagation of the error may constitute a learning cycle (epoch). The learning rate may be applied differently according to the number of repetition times of the learning cycle of the neural network. For example, in an initial stage of the learning of the neural network, the neural network ensures a certain level of performance quickly by using a high learning rate, thereby increasing efficiency and uses a low learning rate in a latter stage of the learning, thereby increasing accuracy.

In learning of the neural network, the learning data may be generally a subset of actual data (i.e., data to be processed using the learned neural network), and as a result, there may be a learning cycle in which errors for the learning data decrease, but the errors for the actual data increase. Overfitting is a phenomenon in which the errors for the actual data increase due to excessive learning of the learning data. For example, a phenomenon in which the neural network that learns a cat by showing a yellow cat sees a cat other than the yellow cat and does not recognize the corresponding cat as the cat may be a kind of overfitting. The overfitting may act as a cause which increases the error of the machine learning algorithm. Various optimization methods may be used in order to prevent the overfitting. In order to prevent the overfitting, a method such as increasing the learning data, regularization, dropout of omitting a part of the node of the network in the process of learning, utilization of a batch normalization layer, etc., may be applied.

In an embodiment of the present disclosure, the variant classification model 300 may be trained by a supervised learning scheme. For example, the variant classification model 300 may be trained based on training data 330 including input data representing whether the mutation of each of the AA mutation identifiers occurs, and ground truth data representing the variant type corresponding to the input data.

In an additional embodiment of the present disclosure, the variant classification model 300 may also be performed based on a weakly supervised learning scheme.

In an embodiment of the present disclosure, a filtering algorithm 320 is applied to raw data 310 to generate the training data 330.

In an embodiment, the raw data 310 may mean data in which the AA mutation information and the variant type are mapped. The raw data 310 may mean data in which the variant type and the AA mutation information are pre-analyzed with respect to a plurality of samples of the target analyte.

In an embodiment, the raw data 310 may include information on at least one of a nucleic acid sequence of the target analyte, and mutation and variant which the corresponding nucleic acid sequence has. Here, the information on the mutation as a base or amino acid level mutation type detected in the corresponding nucleic sequence, may be expressed, for example, as the type of mutation identifier. Further, the information on the variant may include a variant type which the corresponding target analyte is written to have due to the corresponding mutation.

In an embodiment, the raw data 310 may be data obtained from a public data storage, or data processed from the obtained data. As an example, the raw data 310 may include a mutation type and a variant type which the nucleic acid sequence of the target analyte obtained from the public data storage has. As another example, the nucleic acid sequence of the target analyte and the corresponding variant type may be obtained from the public data storage, and mutation types which the corresponding nucleic acid sequence has may be derived through a comparison of the obtained nucleic acid sequence and a representative sequence prestored for the corresponding target analyte, and the raw data 310 may be processed to include the derived mutation types and the corresponding variant type. In another embodiment, the raw data 310 may be obtained by a preprocessing process for the data obtained by the scheme, and in some embodiments, the preprocessing process may include formatting, re-labeling, and encoding disclosed below.

The raw data 310 in the present disclosure may include mutation information for variants of the target analyte obtained from the public data storage such as the National Center for Biotechnology Information (NCBI), and/or Global Initiative on Sharing All Influenza Data (GISAID), for example. As another example, the raw data 310 in the present disclosure may also mean that a format of data having various formats obtained from a plurality of public data storages is formatted. As yet another example, the raw data 310 in the present disclosure may also mean data in which re-labeling of unifying labels of data (e.g., variant type) obtained from the plurality of public data storages is performed. As still yet another embodiment, the raw data 310 in the present disclosure may mean data in which whether the mutation of the AA mutation identifier for the variant type occurs is encoded. Here, encoding may include, for example, one-hot encoding. In an embodiment, whether the mutation of the AA mutation identifier occurs may be expressed as 0 and 1 according to one-hot encoding, and a vector of a dimension corresponding to the number of AA mutation identifiers to which 0 or 1 is allocated may be generated.

In the present disclosure, the filtering algorithm 320 may mean an algorithm for reducing the size of the raw data 310. For example, the filtering algorithm 320 may reduce the size of the AA mutation information of the raw data 310. The raw data 310 may include AA mutation information including AA mutation identifiers and data representing whether the mutation of the AA mutation identifiers occurs, and a variant type 350. Here, as the number of plurality of AA mutation identifiers 340 included in the AA mutation information is larger, the size or dimension of the training data 330 input into the variant classification model 300 may be increased. When the size or dimension of the training data 330 is increased, a significant quantity of computing resources are required in the training process of the variant classification model 300. Accordingly, the filtering algorithm 320 may reduce the number of plurality of AA mutation identifiers 340 in order to reduce the size or dimension of the training data 330.

In an embodiment, the computing device 100 applies a first filtering algorithm for dimension reduction of the training data 330 to the raw data 310 to generate the training data 330 including AA mutation identifiers 340 of a reduced number.

In an embodiment, the computing device 100 may group, based on a predetermined threshold number for the number of mutation occurrence times of each of the plurality of AA mutation identifiers 340 for the variant type 350 in the raw data 310, the plurality of AA mutation identifiers 340 included in the raw data 310 into AA mutation identifiers of a first group which is equal to or more than the threshold number and AA mutation identifiers of a second group which is less than the threshold number. The computing device 100 may make the AA mutation identifiers of the first group except for the AA mutation identifiers of the second group be included in the training data 330 to reduce the size or dimension of the training data 330. Accordingly, the AA mutation identifiers 340 with the number to which the value of 1 is allocated among all samples included in the raw data 310 is less than the threshold number are not included in the training data 330. The threshold number may be determined based on a performance, a computation speed, and/or a computing resource for the classification result of the variant classification model 300. The number of AA mutation identifiers 340 included in the training data 330 may be determined according to the threshold number, and as a result, the dimension of the training data 330 may be determined.

In an additional embodiment, the computing device 100 may also apply the filtering algorithm for each variant type 350. The computing device 100 may align the raw data 310 in respect to the variant type 350. According to the alignment, whether the mutation of each of the AA mutation identifiers 340 occurs may be confirmed for each variant type 350.. The computing device 100 may align AA mutation identifiers 340 in an order in which the number of AA mutation identifiers 340 which are mutated (that is, to which the value of 1 is allocated) is largest (or in an order in which the number is smallest) for each variant type 350 from the obtained raw data 310. Based on the alignment, the computing device 100 may make AA mutation identifiers in which the number of AA mutation identifiers which are mutated is less than a predetermined threshold number for each variant type 350 not be included in the training data 330. The threshold number may be determined based on the performance, the computation speed, and/or the computing resource for the classification result of the variant classification model 300. The number of AA mutation identifiers 340 included in the training data 330 may be determined according to the threshold number, and as a result, the dimension of the training data 330 may be determined.

It will be apparent to those skilled in the art that the above-described threshold number may be expressed as a form of a threshold having a probability value, for example.

Further, it is described that the above-described filtering algorithm compares the number of AA mutation identifiers 340 to which the value of 1 is allocated (that is, of which mutation occurs) among the AA mutation identifiers 340 with the threshold number. According to an aspect of implementation, comparing the number of AA mutation identifiers 340 to which the value of 0 is allocated (that is, of which mutation does not occur) among the AA mutation identifiers 340 with the threshold number will also be included in the scope of the present disclosure.

In an embodiment, multiple variants may be present for the target analyte, and AA mutation identifiers 340 which become a cause of the multiple variants may have numerous numbers. Accordingly, the number of AA mutation identifiers 340 included in the training data 330 may be reduced through the filtering algorithm according to an embodiment of the present disclosure. For example, it is assumed that there is an AA mutation identifier 340 expressed as "spike_N485del". The spike _N485del may have the number of mutation occurrence times corresponding to 2% of the samples included in the raw data 310. As such, since the number of mutation occurrence times for the AA mutation identifier 340 such as spike_N485del is very small (for example, less than 80% which is a threshold) among the raw data 310, the AA mutation identifier 340 may not be included in the training data 330, but may be removed during a filtering process. Since 450 AA mutation identifiers 340 may be included in the training data 330 among thousands of AA mutation identifiers 340 through the filtering process, for example, a training process of the variant classification model 300 may be efficiently conducted as the dimension of the training data 330 is reduced.

In an additional embodiment of the present disclosure, the computing device 100 may perform the filtering algorithm 320 for reducing the number of variant types 350. For example, the computing device 100 may determine the number of samples for each variant type 350 in the raw data 310. The computing device 100 may exclude variant types 350 of which the number is smaller than a threshold number from the training data 330 through the filtering algorithm 320. For example, it is assumed that a variant type 350 expressed as lambda and a variant type 350 expressed as Omega are variant types 350 of which the number is smaller than a threshold number (e.g., 2, 5, and 10) on the raw data 310. The computing device 100 may reduce the number of classes of the variant classification model 300 while reducing the number of raw data 310 by a scheme of excluding data corresponding to these minority classes among the raw data 310 from the training data 330 or granting an integrated label. When the number of classes is reduced, the efficiency in the training process of the variant classification model 300 may also be increased. As an example, the scheme of granting the integrated label may include re-labeling two or more raw data including two or more variant types of which the number is smaller than a predetermined threshold number to one variant type.

In an additional embodiment, the computing device 100 may also reduce the size of the raw data 310 by removing duplicated samples having identical values using the filtering algorithm 320.

In an embodiment, the computing device 100 applies a second filtering algorithm to a first number of raw data 310 to which the AA mutation information is mapped to the variant type 350 to generate training data 330 of a second number which is smaller than the first number. The computing device 100 excludes raw data 310 including variant types 350 of a number smaller than a predetermined threshold number (e.g., 1, 2, 3, 5, 10, 20, etc.) from the training data 330, so computing resources consumed for learning a minority class may be efficiently used.

In an embodiment of the present disclosure, as illustrated in FIG. 3, the training data 330 may be configured by a combination of the variant type 350, and a plurality of AA mutation identifiers 340 to which a value of 0 or 1 is allocated. Here, the plurality of AA mutation identifiers 340 to which the value of 0 or 1 is allocated corresponds to input data, and the variant type 350 corresponding thereto corresponds to ground truth data. In an embodiment, one training data 330 may be expressed as a value allocated to each of the plurality of AA mutation identifiers 340 mapped to one variant type 350, as represented by reference numeral 360. The input data among one training data may mean data vectorized based on the values of 0 and 1 allocated to each of the plurality of AA mutation identifiers. The vectorized data may have, for example, a dimension corresponding to the number of AA mutation identifiers. In this specification, as an example for one-hot encoding, it is exemplified that 0 or 1 is allocated to the AA mutation identifier 340, but according to the aspect of the implementation, a binary value, a ternary value, and/or a quad value may also be allocated to the AA mutation identifier 340.

In an embodiment of the present disclosure, when the training data 330 is input into the variant classification model 300, the variant classification model 300 may output the variant type 350 based on a combination of the values of 0 or 1 allocated to each of the AA mutation identifiers 340 included in the training data 330. An output for the variant type 350 may include one or more variant types 350 corresponding to the input data and scores (e.g., probability values) thereof. In a training process, the variant type 350 included in the output of the variant classification model 300 and the variant type 350 included in the ground truth data of the training data are compared to train the variant classification model 300 by a scheme in which a comparison difference (e.g., a loss function) is minimized.

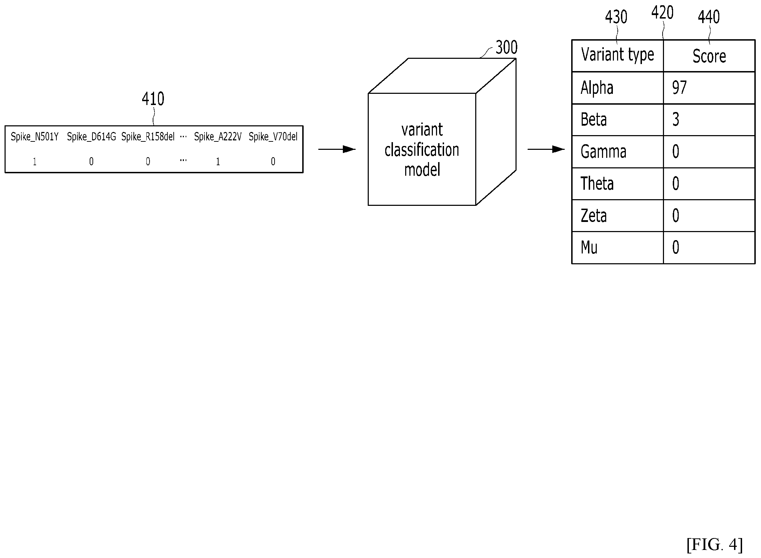

FIG. 4 exemplarily illustrates a conceptual view for an operation of a variant classification model trained according to an embodiment of the present disclosure.

In an embodiment, a process of operating the variant classification model 300 illustrated in FIG. 4 may be performed by the computing device 100. In an additional embodiment, like a scheme in which a part of the process of operating the variant classification model 300 illustrated in FIG. 4 is performed by the user terminal and the other part is performed by the server, the steps in FIG. 4 may also be implemented by a plurality of entities.

The embodiment illustrated in FIG. 4 may include, for example, an inference process of the variant classification model 300.

In an embodiment of the present disclosure, the variant classification model 300 may receive input data 410 and output output data 420. The input data 410 may include binary information allocated to each of the plurality of AA mutation identifiers. In this specification, for convenience of description, it is illustrated that each AA mutation identifier is included in the input data 410, but this is for clarity of the description of the present disclosure, and the AA mutation identifier may be excluded, and respective time series binary values may also be included in the input data 410.

The output data 420 may include a variant type 430 corresponding to the input data 410 and a score for the variant type 430. The score herein may also be expressed as, for example, the probability value or a confidence score.

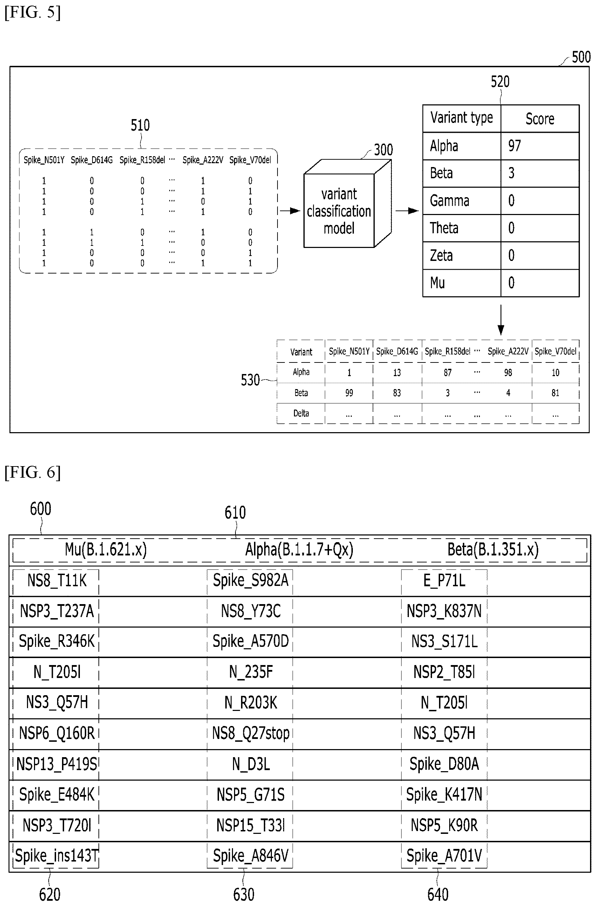

As described below in FIG. 5, the computing device 100 may analyze the output of the variant classification model 300 while changing a value allocated to a predetermined AA mutation identifier of the input data 410.

As illustrated in FIG. 4, since the output data 420 may include the variant type 430 and the score for the variant type 430, the computing device 100 compares a score and a variant type included in output data corresponding to specific input data, and a score and a variant type included in output data corresponding to input data of which some are changed with each other to confirm how large change the changed part in the input data causes in the output data. For example, the change in the output data may include a change in the variant type and/or a change in the score allocated to the variant type.

As an example, the computing device 100 may analyze a change of the output of the variant classification model according to the change in values of the AA mutation identifiers included in the AA mutation information. Based on the change of the output of the variant classification model, the computing device 100 may analyze which AA mutation identifier among the AA mutation identifiers included in the AA mutation information has a high contribution.

FIG. 5 exemplarily illustrates a conceptual view of calculating a contribution of AA mutation identifiers for variant types by using the variant classification model according to an embodiment of the present disclosure.

In an embodiment, a process of calculating the contribution by using the variant classification model 300 illustrated in FIG. 5 may be performed by the computing device 100. In an additional embodiment, like a scheme in which a part of the process of calculating the contribution illustrated in FIG. 5 is performed by the user terminal and the other part is performed by the server, the steps in FIG. 5 may also be implemented by a plurality of entities.

An entity represented by reference numeral 500 in FIG. 5 may mean a model that generates changed input data 510, inputs the changed input data 510 into the variant classification model 300, and calculates a contribution 530 of each of the AA mutation identifiers for each variant type by using output data 520 of the variant classification model 300. The model 500 may include, for example, an explainable AI model. The model 500 that calculates the contribution 530 may calculate the contribution 530 of each of the AA mutation identifiers for each variant type based on a scheme of computing a feature importance of each of the AA mutation identifiers for each variant type.

The explainable AI model 500 may include, for example, an SHAP model which is operable based on the input data and a weight of the variant classification model 300. As an example, the model 500 may mean a model that may calculate a feature importance for each class, and the class herein may correspond to a variant type. Accordingly, since an importance or a contribution of the AA mutation identifiers may be calculated for each specific class, a technique according to an embodiment of the present disclosure may determine what AA mutation identifiers specific to a specific variant type and how much the contribution of the corresponding AA mutation identifier is.

In an embodiment, the model 500 that calculates the contribution may determine, at least partially based on a pre-trained weight of the variant classification model 300, what an AA mutation identifier contributes to outputting a classification result 520 for the variant type is.

In an embodiment, the model 500 that calculates the contribution may determine what AA mutation identifiers having a specific contribution for any one variant type among a plurality of variant types compared to other variant types are, by using the pre-trained variant classification model 300.

In an embodiment, the model 500 may calculate the contribution based on a difference between a plurality of mutation information and a difference between variant types obtained from the variant classification model 300 due to the corresponding difference. Here, the difference between the plurality of mutation information represents a difference between input data of the variant classification model 300, and may include, for example, a difference in value (e.g., whether mutation occurs, 0 or 1) between input data among a plurality of mutation identifiers and the type of mutation identifier having the corresponding difference in value. As an example, a difference between first mutation information and second mutation information may be obtained through a difference between a value of a first vector included in the first mutation information and a value of a second vector included in the second mutation information. Further, the difference between the variant types obtained from the variant classification model 300 due to the difference between the plurality of mutation information represents a difference between output data obtained from the variant classification model 300 due to a difference between the input data of the variant classification model 300, and may include, for example, a difference (e.g., whether the variant type is changed and a change type) in variant type obtained from the variant classification model 300 due to a difference in value between mutation identifiers.

In an embodiment, the model 500 may calculate the contribution 530 of a specific AA mutation identifier by computing a difference between output data for input data which is not changed and input data of changing a value of the AA mutation identifierFor example, the model 500 compares a first classification result output from the variant classification model 300 in response to first input data of changing the value of a first AA mutation identifier among the plurality of AA mutation identifiers included in the AA mutation information and a second classification result output from the variant classification model 300 in response to second input data of not changing the value of the first AA mutation identifier among the plurality of AA mutation identifiers, for a first variant type among the plurality of variant types to calculate a contribution of the first AA mutation identifier for the first variant type.

In such an example, the contribution of the first AA mutation identifier for the first variant type may have a higher value as a difference value between the first classification result and the second classification result is larger. That is, when a change amount of a result output as the value of the first AA mutation for the first variant type is changed is large, it may be determined that the feature importance of the first AA mutation for the first variant type is high.

In such an example, the contribution of the first AA mutation identifier for the first variant type may have a lower value as a difference value between a first classification result when the value of the first AA mutation is changed and a second classification result when the value of the first AA mutation is not changed is larger, for a second variant type different from the first variant type. In such a case, an AA mutation identifier having a high contribution for another variant type different from the first variant type may not be considered an AA mutation identifier specific to the first variant type. Accordingly, the contribution of the first AA mutation identifier for the first variant type may be computed additionally based on the contribution of the first AA mutation identifier for another variant type.

The input data 510 in the present disclosure may be used as a meaning that encompasses input data of changing a value corresponding to at least a part of the input data to a different value and/or input data which is not changed. The input data may include training data used in the training process of the variant classification model 300. In another embodiment, the input data may include data different from the training data used in the training process of the variant classification model 300.

In an embodiment of the present disclosure, the input data 510 may include perturbation input data. For example, perturbation of the input data may determine an AA mutation identifier to be changed in the input data by a random scheme. As another example, the perturbation of the input data may also determine an AA mutation identifier to be changed in the input data according to a predetermined order (e.g., a time series order of the input data).

According to an embodiment of the present disclosure, the model 500 may output a degree to contribute to determining each variant type for each AA mutation identifier by using perturbation input data generated while changing at least a part of the input data.

In an embodiment, the perturbation for the input data may mean changing a value of a specific AA mutation identifier in the input data to another value. As an example, the perturbation input data may mean input data of the value of the specific AA mutation identifier changed from 0 to 1 or from 1 to 0. According to the implementation aspect, the perturbation for the input data may include changing values of a plurality of AA mutation identifiers or changing a value of one AA mutation identifier. In an embodiment, when perturbation input data is used, which changes the value of the specific AA mutation identifier and fixes values of other AA mutation identifiers, the model 500 analyzes a change of output data 520 corresponding to each of perturbation input data 510 to determine what influence the specific AA mutation identifier exerts on the output data.

In an embodiment of the present disclosure, the perturbation of the input data may also include a permutation operation of substituting at least a part of the input data.

In an additional embodiment of the present disclosure, the perturbation input data may include two input data including a case where the value of the AA mutation identifier is 0 and a case where the value of the AA mutation identifier is 1.

In an embodiment of the present disclosure, the output data 520 obtained in response to the perturbation input data 510 may include information on the variant type and a score corresponding to the variant type. Accordingly, the model 500 may calculate the contribution 530 of the AA mutation identifier for each variant by analyzing the variant type and the score of the output data 520 changed in response to the perturbation input data 510.

For example, the model 500 may express the output data 520 including the variant classification result of the variant classification model 300 generated in response to the specific input data 510 as a form 530, *'since 501st AA of Spike protein is mutated from asparagine to tyrosin, and 222nd AA is mutated from alanine to valine, the input data corresponds to Alpha variant at a probability of 99%*'*.* Accordingly, the contribution of each of the AA mutation identifiers for each variant may be calculated based on the output data 520 of the model 500.

In an embodiment of the present disclosure, the model 500 may calculate a first feature value when mutation occurs in a second AA mutation identifier among the plurality of AA mutation identifiers included in the AA mutation information and a second feature value when mutation does not occur in the second AA mutation identifier. The model 500 may calculate the contribution of the second AA mutation identifier at least partially based on the first feature value and the second feature value. In the above-described example, it may be considered that as a difference between the first feature value and the second feature value is larger, the contribution for the second AA mutation identifier is higher. When a value of a specific AA mutation identifier is changed, the difference of the output feature value being larger may indicate that the corresponding AA mutation identifier is used as an important factor when calculating the output. Accordingly, the difference between these feature values may be associated with the feature importance on the output data.

The term feature importance used in the present disclosure may be associated with an influence of a specific feature in generating the output data of the variant classification model 300. Such feature importance may be used for calculating a contribution for a specific variant type of an AA mutation identifier corresponding to the specific feature. The feature importance in the present disclosure may be used interchangeably with the contribution and/or a feature value contribution.

In an embodiment of the present disclosure, the model 500 compares a third classification result output from the variant classification model 300 in response to third input data corresponding to a third AA mutation identifier among the plurality of AA mutation identifiers included in the AA mutation information and a fourth classification result output from the variant classification model 300 in response to fourth input data corresponding to a fourth AA mutation identifier among the plurality of AA mutation identifiers included in the AA mutation information, for the same variant type to calculate a contribution of at least one of the third AA mutation identifier and the fourth AA mutation identifier for the same variant type.

As described above, the model 500 may calculate a contribution 530 of each of different AA mutation identifiers for the same variant type by using (perturbation) input data 510 of each of different AA mutation identifiers for the same variant type. As an example, the contribution 530 may be output by a comparable scheme in an order of a highest value or a lowest value for each of different AA mutation identifiers.

In an additional embodiment of the present disclosure, the model 500 may also calculate, based on a classification result obtained in response to perturbation input data of changing whether mutation of a specific AA mutation is made for different variants, a contribution 530 of the specific AA mutation identifier for each of variants. As an example, a first difference value of classification results obtained in response to perturbation input data for a case where the first AA mutation identifier is mutated and a case where the first AA mutation identifier is not mutated for a first variant may be calculated. In addition, a second difference value of classification results obtained in response to perturbation input data for a case where the first AA mutation identifier is mutated and a case where the first AA mutation identifier is not mutated for a second variant may be calculated. The model 500 compares the first difference value and the second difference value to determine contributions for the first variant and the second variant of the first AA mutation identifier. When it is determined that the first difference value is larger than the second difference value, an additional weight according to a difference between the first difference value and the second difference value may be set. In such an example, it may be determined that the contribution of the first AA mutation identifier to the first variant is relatively high and the contribution to the second variant is relatively low.

As described above, the model 500 may obtain a fifth classification result output from the variant classification model 300 in response to fifth input data of changing a value of a fifth AA mutation identifier among the plurality of AA mutation identifiers included in the AA mutation information and a sixth classification result output from the variant classification model 300 in response to sixth input data of not changing the value of the fifth AA mutation identifier among the plurality of AA mutation identifiers included in the AA mutation information, for each of at least two variant types. The model 500 compares the fifth classification result and the sixth classification result for each of at least two variant types to calculate the contribution of the fifth AA mutation identifier to each of the at least two variant types.

In an embodiment of the present disclosure, the model 500 may calculate the contribution of specific AA mutation identifier to a specific variant at least partially based on a difference of classification results for a case of changing a value of specific AA mutation for thespecific variant and a case of not changing the value of the specific AA mutation, and the contribution of the specific AA mutation identifier to another variant. For example, the model 500 may calculate the contribution of a specific AA mutation identifier to a specific variant type based on a first factor indicating a classification result obtained from the variant classification model 300 by changing whether a mutation of the specific AA mutation identifier for the specific variant type occurs, and a second factor indicating the contribution of the specific AA mutation identifier calculated for a variant type different from the specific variant type.

FIG. 6 illustrates an exemplary data structure 600 including contribution of AA mutation identifiers for variant types according to an embodiment of the present disclosure.

As illustrated in FIG. 6, an exemplary data structure 600 including contribution of AA mutation identifiers to variant types may include a table structure in which variants 610 constitute a row and AA mutation identifiers 620, 630, and 640 corresponding to the variants, respectively constitute a column. The data structure 600 illustrated in FIG. 6 is an exemplary structure for convenience of description, and any type of data structure which may express the contribution of each of the AA mutation identifiers for each of the variants may also be included in the scope of the present disclosure according to the implementation aspect.

As illustrated in FIG. 6, for a Mu variant, it may be determined that a contribution of an AA mutation identifier expressed as NS8_T11K is highest, and a contribution of an AA mutation identifier expressed as NSP3_T237A is second highest. As such, AA mutation identifiers 620 may be listed in an order of a highest contribution (or in an order of a lowest contribution) for the Mu variant. In a process of determining AA mutation identifiers 620 related to a specific contribution for the Mu variant, AA mutation identifiers 630 or 640 determined to have a high contribution in a variant different from the Mu variant(e.g., Alpha variant or Beta variant) may be calculated to have a low weight or may be excluded.

As described above, the technique according to an embodiment of the present disclosure may output, for each variant type of a target analyte, a degree to contribute to determining the corresponding variant type for each AA mutation identifier. The AA mutation identifiers output this way may be results considering both whether the output AA mutation identifiers are specific to the corresponding variant type and the degree to contribute to determining the corresponding variant type. As a result, the technique according to an embodiment of the present disclosure may efficiently calculate, among AA mutation identifiers caused by a specific variant, an AA mutation identifier or a nucleic acid sequence having a high specificity for the corresponding variant.

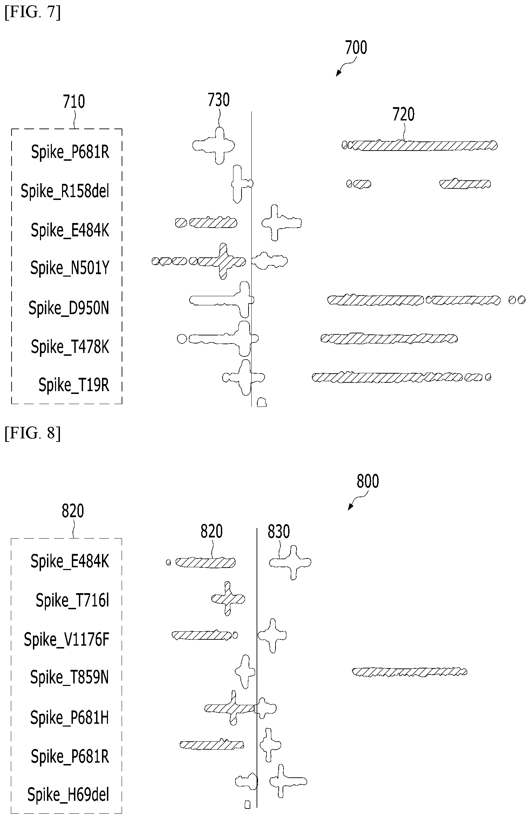

FIG. 7 exemplarily illustrates a data structure 700 for visually showing the contribution of AA mutation identifiers for variant types according to an embodiment of the present disclosure.

In an example of FIG. 7, an importance or a contribution of AA mutation identifiers specific to a Delta variant may be visually expressed.

The data structure 700 illustrated in FIG. 7 represents an example for the output of the model 500 in FIG. 5.

Graphs illustrated in FIG. 7 show concatenated values determined in response to respective samples (e.g., respective perturbation input data).

In an embodiment, left and right ranges in the graph may be used for indicating whether a specificity in a specific class is high. For example, heading to the right may indicate that the specificity is high in the specific class, and heading to the left may indicate that the specificity is low in the specific class. Two types of graphs 720 and 730, i.e., a graph with a hatched line expressed by reference numeral 720 and a graph with no hatched line expressed by reference numeral 730 are shown in FIG. 7. The two types of graphs may show a contribution in a corresponding class according to whether mutation of each of the AA mutation identifiers 710 occurs. The graph expressed by reference numeral 720 shows a contribution when mutation occurs and the graph expressed by reference numeral 730 shows a contribution when mutation does not occur.

In an embodiment, a value of the graph may indicate that the importance or contribution for the corresponding class (e.g., Delta variant) is lower toward the left.

In an embodiment, the value of the graph may indicate that the specificity for the corresponding class (e.g., Delta variant) is lower toward the left. For example, the value of the graph may indicate that the specificity or contribution for another class (e.g., another variant type) is higher toward the left. For example, the value of the graph may mean that there is a high possibility that the AA mutation identifier will not be specific to the corresponding variant type (e.g., Delta variant) toward the left. On the contrary, the value of the graph may mean that there is a high possibility that the AA mutation identifier will be specific to the corresponding variant type (e.g., Delta variant) toward the right.

The graph expressed by reference numeral 720 relates to the case where the value of the AA mutation identifier is 1, and the graph expressed by reference numeral 730 relates to the case where the value of the AA mutation identifier is 0. That is, the graph expressed by reference numeral 720 relates to the case where the mutation of the AA mutation identifier occurs, and the graph expressed by reference numeral 730 relates to the case where the mutation of the AA mutation identifier does notoccur.

For example, as the value of the graph expressed by reference numeral 720 goes to the right, it indicates that there is a high possibility of the Delta variant when the mutation of the AA mutation identifier occurs. That is, as the value of the graph expressed by reference numeral 720 goes to the right, it indicates that there is a high possibility of the Delta variant when the value of the AA mutation identifier is 1.

For example, as the value of the graph expressed by reference numeral 730 goes to the left, it indicates that there is a high possibility of the Delta variant when the mutation of the AA mutation identifier does not occur. That is, as the value of the graph expressed by reference numeral 730 goes to the left, it indicates that there is a high possibility that there will be the Delta variant when the value of the AA mutation identifier is 0.

For example, when the mutation of an AA mutation identifier expressed as Spike_P681R occurs, there may be a high probability of the Delta variant. Accordingly, referring to the example illustrated in FIG. 7, it may be construed that the Delta variant is generated as AA mutation identifiers corresponding to Spike _P681R, Spike _R158del, Spike_D950N, Spike_T478K, and Spike_T19R are mutated. Accordingly, it may be determined that a specificity and a contribution of mutation of Spike _P681R, Spike_R158del, Spike_D950N, Spike_T478K, and Spike_T19R to the Delta variant are high. Accordingly, in calculating the specificity and the contribution to the Delta variant, Spike_P681R, Spike_R158del, Spike_D950N, Spike_T478K, and Spike_T19R may have a high weight.

As another example, there is a high possibility of Delta variant only ifthe mutation of AA mutation identifiers corresponding to Spike_E484K and Spike_N501Y does not occur. Further, there is a high possibility that the mutation of AA mutation identifiers corresponding to Spike_E484K and Spike_N501Y occurs in other variants. Accordingly, in calculating the specificity and the contribution to the Delta variant, Spike_E484K and Spike_N501Y may have a low weight.

In an embodiment, the computing device 100 may calculate specific contributions of the Delta variant for respective AA mutation identifiers based on the values of the graphs represented by reference numeral 720. For example, the computing device 100 may calculate the specific contributions of the Delta variant for the respective AA mutation identifiers by aggregating or averaging the values of the graphs represented by reference numeral 720.

In an additional embodiment, the computing device 100 may calculate specific contributions of the Delta variant for respective AA mutation identifiers additionally based on the values of the graphs represented by reference numeral 730. For example, the computing device 100 may calculate the specific contributions of the Delta variant for the respective AA mutation identifiers by subtracting the values of the graphs represented by reference numeral 730.

In an additional embodiment, the computing device 100 may calculate specific contributions of the Delta variant for respective AA mutation identifiers based on values of graphs corresponding to a right side of a predefined reference line among the values of the graphs represented by reference numeral 720.

In an embodiment of the present disclosure, the computing device 100 may calculate a contribution of each of the plurality of AA mutation identifiers 710 for each of the plurality of variant types so that each of the plurality of the AA mutation identifiers 710 has a positive influence value or a negative influence value.

As an example, the positive influence herein may correspond to reference numeral 720, and the negative influence may correspond to reference numeral 730. For example, the positive influence may indicate how much, when the value of the AA mutation identifier is 1, the value influences determining a specific variant type, and the negative influence may indicate how much, when the value of the AA mutation identifier is 0, the value influences determining a specific variant type. The value of the graph may indicate that the positive influence and the negative influence are larger toward the right, and the value of the graph may indicate that the positive influence and the negative influence are smaller toward the left.

As another example, the positive influence and the negative influence may also be used for meaning a value corresponding to an X axis on the graph. In such an example, the positive influence may be larger toward the right based on a predefined reference value for a value of the X axis on the graph, and the negative influence is larger toward the left.

FIG. 8 exemplarily illustrates a data structure 800 for visually showing the contribution of AA mutation identifiers for variant types according to an embodiment of the present disclosure.

In an example of FIG. 8, an importance or a contribution of AA mutation identifiers specific to a Lambda variant may be visually expressed. A part in a description of FIG. 8 which is duplicated with the description of FIG. 7 will be omitted for simplification of the description.

As described above in FIG. 7, graphs of two types 820 and 830 illustrated in FIG. 8 may show a contribution of a corresponding class according to whether mutation of each of AA mutation identifiers 810 occurs. The graph expressed by reference numeral 820 shows a contribution when mutation occurs and the graph expressed by reference numeral 830 shows a contribution when mutation does not occur.

As described above in FIG. 7, a value of the graph illustrated in FIG. 8 may indicate that the specificity or contribution for the corresponding class (e.g., Lambda variant) is lower toward the left.

In an example of FIG. 8, when a value for an AA mutation identifier expressed by Spike_T859N is 1 (That is, the mutation occurs), the AA mutation identifier may have a high specificity and/or contribution to the Lambda variant.

As another example, there is a high possibility that mutation for AA mutation identifiers expressed as Spike_E484K, Spike_T716I, Spike _V1176F, Spike_P681H, and SpikeP681R will occur for other variants different from the Lambda variant. Accordingly, it may be evaluated that the AA mutation identifiers expressed as Spike_E484K, Spike_T716I, Spike _V1176F, Spike_P681H, and SpikeP681R have a lower specificity and/or contribution to the Lambda variant.

As an additional example, when the values for the AA mutation identifiers expressed as Spike_E484K, Spike_T716I, Spike_V1176F, Spike_P681H, and SpikeP681R are 0, there is a high possibility that it will belong to the Lambda variant. In this case, the values for the AA mutation identifiers expressed as Spike_E484K, Spike_T716I, Spike_V1176F, Spike_P681H, and SpikeP681R being 0 may be related to a high specificity and/or contribution to the Lambda variant.

As illustrated in FIGS. 7 and 8, the technique according to an embodiment of the present disclosure may determine an AA mutation identifier which is specific to a specific variant type and has a high contribution to the variant type by an efficient scheme. Accordingly, based on a feature importance for each class considering the contribution to the other variant types, the AA mutation identifier which has the high contribution may be efficiently and accurately computed for each variant.

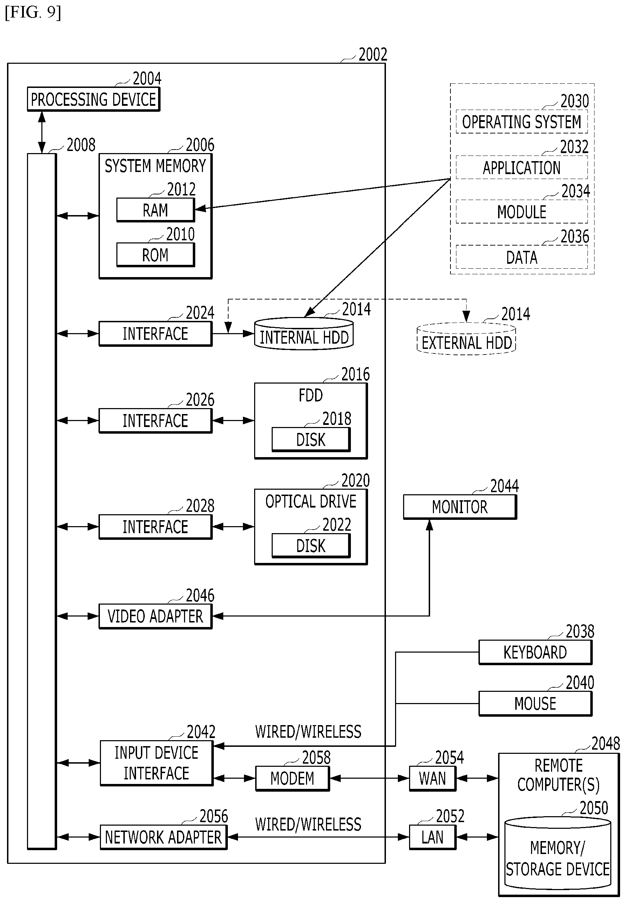

FIG. 9 is a schematic diagram of a computing environment according to an embodiment of the present disclosure.

In the present disclosure, a component, a module or a unit includes a routine, a procedure, a program, a component, a data structure, and the like that perform a specific task or implement a specific abstract data type. Further, it will be well appreciated by those skilled in the art that the method of the present disclosure can be implemented by other computer system configurations including a personal computer, a handheld computing device, microprocessor-based or programmable home appliances, and others (the respective devices may operate in connection with one or more associated devices) as well as a single-processor or multi-processor computer system, a mini computer, and a main frame computer.

The exemplary embodiments described in the present disclosure may also be implemented in a distributed computing environment in which predetermined tasks are performed by remote processing devices connected through a communication network. In the distributed computing environment, the program module may be positioned in both local and remote memory storage devices.

The computer generally includes various computer readable media. The computer includes, as a computer accessible medium, volatile and non-volatile media, transitory and non-transitory media, and mobile and non-mobile media. As a non-limiting example, the computer readable media may include both computer readable storage media and computer readable transmission media.

The computer readable storage media include volatile and non-volatile media, transitory and non-transitory media, and mobile and non-mobile media implemented by a predetermined method or technology for storing information such as a computer readable instruction, a data structure, a program module, or other data. The computer readable storage media include a RAM, a ROM, an EEPROM, a flash memory or other memory technologies, a CD-ROM, a digital video disk (DVD) or other optical disk storage devices, a magnetic cassette, a magnetic tape, a magnetic disk storage device or other magnetic storage devices or any other media which may be accessed by the computer or may be used to store desired information, but are not limited thereto.

The computer readable transmission media generally implement the computer readable instruction, the data structure, the program module, or other data in a carrier wave or a modulated data signal such as other transport mechanism and include all information transfer media. The term "modulated data signal" means a signal acquired by setting or changing at least one of characteristics of the signal so as to encode information in the signal. As a non-limiting example, the computer readable transmission media include wired media such as a wired network or a direct-wired connection and wireless media such as acoustic, RF, infrared and other wireless media. A combination of any media among the aforementioned media is also included in the computer readable transmission media.

An exemplary environment 2000 that implements various aspects of the present disclosure including a computer 2002 is shown and the computer 2002 includes a processing device 2004, a system memory 2006, and a system bus 2008. The system bus 2008 connects system components including the system memory 2006 (not limited thereto) to the processing device 2004. The processing device 2004 may be a predetermined processor among various commercial processors. A dual processor and other multi-processor architectures may also be used as the processing device 2004.

The system bus 2008 may be any one of several types of bus structures which may be additionally interconnected to a local bus using any one of a memory bus, a peripheral device bus, and various commercial bus architectures. The system memory 2006 includes a read only memory (ROM) 2010 and a random access memory (RAM) 2012. A basic input/output system (BIOS) is stored in the non-volatile memories 2010 including the ROM, the EPROM, the EEPROM, and the like and the BIOS includes a basic routine that assists in transmitting information among components in the computer 2002 at a time such as in-starting. The RAM 2012 may also include a high-speed RAM including a static RAM for caching data, and the like.

The computer 2002 also includes an internal hard disk drive (HDD) 2014 (for example, EIDE and SATA) - the internal hard disk drive 2014 may also be configured for an external purpose in an appropriate chassis (not illustrated), a magnetic floppy disk drive (FDD) 2016 (for example, for reading from or writing in a mobile diskette 2018), and an optical disk drive 2020 (for example, for reading a CD-ROM disk 2022 or reading from or writing in other high-capacity optical media such as the DVD). The hard disk drive 2014, the magnetic disk drive 2016, and the optical disk drive 2020 may be connected to the system bus 2008 by a hard disk drive interface 2024, a magnetic disk drive interface 2026, and an optical disk drive interface 2028, respectively. An interface 2024 for implementing an external drive includes, for example, at least one of a universal serial bus (USB) and an IEEE 1394 interface technology or both of them.

The drives and the computer readable media associated therewith provide non-volatile storage of the data, the data structure, the computer executable instruction, and others. In the case of the computer 2002, the drives and the media correspond to storing of predetermined data in an appropriate digital format. In the description of the computer readable storage media, the mobile optical media such as the HDD, the mobile magnetic disk, and the CD or the DVD are mentioned, but it will be well appreciated by those skilled in the art that other types of storage media readable by the computer such as a zip drive, a magnetic cassette, a flash memory card, a cartridge, and others may also be used in an exemplary operating environment and further, the predetermined media may include computer executable instructions for executing the methods of the present disclosure.

Multiple program modules including an operating system 2030, one or more application programs 2032, other program module 2034, and program data 2036 may be stored in the drive and the RAM 2012. All or some of the operating system, the application, the module, and/or the data may also be cached in the RAM 2012. It will be well appreciated that the present disclosure may be implemented in operating systems which are commercially usable or a combination of the operating systems.

A user may input instructions and information in the computer 2002 through one or more wired/wireless input devices, for example, pointing devices such as a keyboard 2038 and a mouse 2040. Other input devices (not illustrated) may include a microphone, an IR remote controller, a joystick, a game pad, a stylus pen, a touch screen, and others. These and other input devices are often connected to the processing device 2004 through an input device interface 2042 connected to the system bus 2008, but may be connected by other interfaces including a parallel port, an IEEE 1394 serial port, a game port, a USB port, an IR interface, and others.

A monitor 2044 or other types of display devices are also connected to the system bus 2008 through interfaces such as a video adapter 2046, and the like. In addition to the monitor 2044, the computer generally includes other peripheral output devices (not illustrated) such as a speaker, a printer, others.

The computer 2002 may operate in a networked environment by using a logical connection to one or more remote computers including remote computer(s) 2048 through wired and/or wireless communication. The remote computer(s) 2048 may be a workstation, a server computer, a router, a personal computer, a portable computer, a micro-processor based entertainment apparatus, a peer device, or other general network nodes and generally includes multiple components or all of the components described with respect to the computer 2002, but only a memory storage device 2050 is illustrated for brief description. The illustrated logical connection includes a wired/wireless connection to a local area network (LAN) 2052 and/or a larger network, for example, a wide area network (WAN) 2054. The LAN and WAN networking environments are general environments infices and companies and facilitate an enterprise-wide computer network such as Intranet, and all of them may be connected to a worldwide computer network, for example, the Internet.

When the computer 2002 is used in the LAN networking environment, the computer 2002 is connected to a local network 2052 through a wired and/or wireless communication network interface or an adapter 2056. The adapter 2056 may facilitate the wired or wireless communication to the LAN 2052 and the LAN 2052 also includes a wireless access point installed therein order to communicate with the wireless adapter 2056. When the computer 2002 is used in the WAN networking environment, the computer 2002 may include a modem 2058, is connected to a communication server on the WAN 2054, or has other means that configure communication through the WAN 2054 such as the Internet, etc. The modem 2058 which may be an internal or external and wired or wireless device is connected to the system bus 2008 through the serial port interface 2042. In the networked environment, the program modules described with respect to the computer 2002 or some thereof may be stored in the remote memory/storage device 2050. It will be well known that an illustrated network connection is exemplary and other means configuring a communication link among computers may be used.

The computer 2002 performs an operation of communicating with predetermined wireless devices or entities which are disposed and operated by the wireless communication, for example, the printer, a scanner, a desktop and/or a portable computer, a portable data assistant (PDA), a communication satellite, predetermined equipment or place associated with a wireless detectable tag, and a telephone. This at least includes wireless fidelity (Wi-Fi) and Bluetooth wireless technology. Accordingly, communication may be a predefined structure like the network in the related art or just ad hoc communication between at least two devices.

It shall be understood that a specific order or a hierarchical structure of the operations included in the presented processes is an example of illustrative accesses. It shall be understood that a specific order or a hierarchical structure of the operations included in the processes may be rearranged within the scope of the present disclosure based on design priorities. The accompanying method claims provide various operations of elements in a sample order, but it does not mean that the claims are limited to the presented specific order or hierarchical structure.

### [Mode for Invention]

Related contents in the best mode for carrying out the present disclosure are described as above.

### [Industrial Applicability]

The present disclosure may be used in the device, the system, etc., which analyze the variant type of the organism.

## Claims

1. A method for analyzing a variant type of an organism, performed by a computing device, the method comprising:
preparing a variant classification model trained with a plurality of datasets, each of the plurality of datasets including (i) a mutation information including at least one mutation identifier and (ii) a variant type labeled thereto;
providing a plurality of mutation information to the variant classification model to obtain variant types for each of the plurality of mutation information; and
calculating a contribution of said at least one mutation identifier to determining each of the variant types obtained, based on the plurality of mutation information and each of the variant types obtained.

2. The method of claim 1, wherein the analyzing the variant type of the organism comprises the obtaining of the variant types for the plurality of mutation information; and
the calculating of the contribution.

3. The method of claim 1, wherein the variant classification model is a multi class classification model pre-trained based on at least one selected from the group consisting of Random forest, KNN (K-nearest neighbor), Naive bayes or MLP (multi-layer perceptron), and
wherein one class corresponds to one variant type.

4. The method of claim 1, wherein the at least one mutation identifier permits to identify mutated and/or non-mutated amino acids.

5. The method of claim 1, wherein the mutation information is vectorized into a dimension corresponding to a count of the mutation identifiers.

6. The method of claim 1, wherein the plurality of datasets used for the training is the datasets subjected to a dimension reduction algorithm to reduce a count of a plurality of the mutation identifiers included in the mutation information.

7. The method of claim 1, wherein the plurality of datasets used for the training are datasets subjected to a filtering algorithm which removes one or more datasets based on a count of mutation occurrences.

8. The method of claim 1, wherein the contribution is calculated based on a difference between the plurality of mutation information and a difference between the variant types obtained from the variant classification model due to the difference between the plurality of mutation information.

9. The method of claim 1, wherein the contribution is calculated using explainable AI (artificial intelligence) calculating a feature importance of each of the mutation identifiers for each of the variant types in the variant classification model.

10. The method of claim 1, further comprising :
generating perturbation input data by changing at least one of the plurality of mutation information; and
providing the perturbation input data to the variant classification model to obtain a variant type;
wherein the perturbation input data and the variant type obtained therefrom are additionally used for calculating the contribution.

11. The method of claim 1, further comprising :
calculating, based on the calculated contribution, a specificity ranking of the plurality of mutation identifiers for each of the variant types obtained.

12. The method of claim 1, wherein the calculating the contribution comprises :
calculating the contribution of each of the plurality of mutation identifiers for each of the variant types obtained in such a manner that, each of the plurality of mutation identifiers included in the mutation information is allowed to comprise a positive influence value or a negative influence value.

13. A computing device comprising:
a memory comprising at least one instruction; and
a processor, and
as the at least one instruction is executed by the processor:
a variant classification model trained with a plurality of datasets is prepared, each of the plurality of datasets including (i) a mutation information including at least one mutation identifier and (ii) a variant type labeled thereto;
a plurality of mutation information is provided to the variant classification model to obtain variant types for each of the plurality of mutation information; and
a contribution of said at least one mutation identifier to determining each of the variant types obtained is calculated, based on the plurality of mutation information and each of the variant types obtained.

14. A computer-readable recording medium on which a computer program is stored, wherein the computer program is programmed to perform each of steps included in the method of claim 1.
